# EUROPEAN PATENT APPLICATION

(11) **EP 3 279 318 A1**
(43) Date of publication of application: **07.02.2018**
(21) Application number: 16773060.5
(22) Date of filing: 30.03.2016
(51) Int. Cl.: C12N 5/0797

(54) **HUMAN SERUM ALBUMIN-CONTAINING CULTURE MEDIUM FOR GROWTH OF NEURAL STEM CELLS**

(30) Priority: 30.03.2015 JP 2015069979
(71) Applicant: Ajinomoto Co., Inc., Tokyo 104-8315 (JP)
(72) Inventor: MATSUMOTO, Takuya, Kawasaki-shi Kanagawa 210-8681 (JP); SENDA, Sho, Kawasaki-shi Kanagawa 210-8681 (JP); KOBAYASHI, Tsuyoshi, Kawasaki-shi Kanagawa 210-8681 (JP)
(74) Representative: Hoffmann Eitle
(86) International application number: PCT/JP2016/060554
(87) International publication number: WO 2016/159179

(57) **Abstract**

The present invention provides a medium for proliferating neural stem cells and/or neural progenitor cells, which contains human serum albumin, and promotes cell proliferation of neural stem cells and/or neural progenitor cells while maintaining undifferentiated state and multipotency, a method of proliferating neural stem cells and/or neural progenitor cells by using the medium, and the like.

## Description

### [Technical Field]

### [Technical Field of invention]

The present invention relates to a growth medium for neural stem cells and/or neural progenitor cells, which contains human serum albumin, and a growth method of neural stem cells and/or neural progenitor cells, which comprises using the medium, and the like.

### [Background Art]

Neural stem cell is an undifferentiated cell having self-replication competence and multipotency, and is capable of generating various cells in the nervous system (nerve cells and neural progenitor cells, glial cells (astrocytes, oligodendrocytes, etc.), glial progenitor cells and the like). Since neural stem cells and neural progenitor cells can supply cells such as nerve cells and the like that are difficult to proliferate in normal adults, it is drawing attention as a source of biomaterials in regenerative medicine, and is expected to be applicable to the treatment of intractable neurological diseases such as amyotrophic lateral sclerosis, Alzheimer's disease, Parkinson's disease and the like, and nerve damage. Since treatments of intractable neurological diseases and nerve damage by using such neural stem cells and/or neural progenitor cells, research and development of treatment methods therefor and the like require a large amount of neural stem cells and/or neural progenitor cells, the development and improvement of a culture method of neural stem cells and/or neural progenitor cells in vitro is one of the important objects.

As culture methods of neural stem cells and/or neural progenitor cells, some methods have been reported to date.

Non-patent document 1 describes neurosphere culture as an in vitro culture method of neural stem cells. This document shows that neural stem cells can be proliferated while maintaining an undifferentiated state of the neural stem cells and maintaining multipotency by culturing the neural stem cells in suspension in a serum-free medium containing epithelial cell growth factor (EGF) and basic fibroblast growth factor (bFGF).

As a method of adherent monolayer culture of neural stem cells and/or neural progenitor cells, a method of culturing neural stem cells and/or neural progenitor cells in a medium containing EGF and/or bFGF on an incubator coated with substrates such as laminin, Poly-L-ornithine, fibronectin and the like, and the like can be mentioned (non-patent document 2).

It has been reported that neural stem cells undergo symmetrical division and self-replicate in the above-mentioned adherent monolayer culture, and the culture is advantageous in that it can provide a uniform cell population as compared to neurosphere culture.

While these culture methods are highly advantageous in that they can culture neural stem cells and neural progenitor cells in vitro, they have a disadvantage that cell proliferation is slow and culturing takes time. Therefore, the development of an improved medium and culture conditions for promoting proliferation of neural stem cells and neural progenitor cells while maintaining undifferentiated state and multipotency thereof is desired.

Heretofore, there have been presented some descriptions regarding a medium containing serum albumin. Patent document 1 discloses a medium in which osmotic pressure is adjusted as a medium for differentiation of a stem cell into an endodermal progenitor cell, and describes that the efficiency of endoderm differentiation induction is promoted when a human induced pluripotent stem (iPS) cell line is cultured in a medium added with human-serum albumin. In non-patent document 3, to study the effect of the final glycation resultant product (AGE) of a glycation reaction (Maillard's reaction) on the differentiation of neural stem cells, neural stem cells are cultured in a medium containing a bovine serum albumin protein modified with AGE. It has been reported that by culturing them in this medium by neurosphere culture, differentiation into astrocyte, which is one kind of glial cell, is promoted. Furthermore, non-patent document 4 describes a medium for the culture of primary brain tumor cells, which is added with bovine serum albumin for the maintenance of osmotic pressure and retention of growth factor and fatty acid. In addition to these, bovine serum albumin is sometimes added to a medium to adjust the osmotic pressure in the medium or solubilize poorly-soluble compounds such as fatty acid and the like, since it is easily available at a low cost.

However, an influence of human serum albumin on the maintenance of undifferentiated state and multipotency of neural stem cells and/or neural progenitor cells and promotion of proliferation thereof has not been clarified at all.

### [Document List]

### [Patent documents]

patent document 1: US patent application publication 20130224857

### [non-patent documents]

non-patent document 1: Science, 1992, 255(5052), 1707-10.
non-patent document 2: PLoS Biology, 2005, 3(9), e283
non-patent document 3: International Journal of Molecular Sciences, 2014, 15(1), 159-170
non-patent document 4: BioTechniques, 2013, 55(2), 83, 85-86, 88

### [SUMMARY OF THE INVENTION]

### [Problems to be Solved by the Invention]

An object of the present invention is to provide a medium for promoting proliferation of neural stem cells and/or neural progenitor cells while maintaining undifferentiated state and multipotency thereof, and also to provide a method of promoting cell proliferation of neural stem cells and/or neural progenitor cells while maintaining undifferentiated state and multipotency thereof.

### [Means of Solving the Problems]

The present inventors have conducted intensive studies in an attempt to achieve the above-mentioned objects, and found that human serum albumin acts to maintain undifferentiated state and multipotency of neural stem cells and/or neural progenitor cells and promote proliferation thereof, which resulted in the completion of the present invention.

That is, the present invention is as follows.
[1] A medium for neural stem cells and/or neural progenitor cells, comprising human serum albumin.
[2] The medium of the above-mentioned [1], which is for maintaining undifferentiation of neural stem cells and/or neural progenitor cells.
[3] The medium of the above-mentioned [1] or [2], which is for promoting proliferation of neural stem cells and/or neural progenitor cells.
[4] The medium of any of the above-mentioned [1] - [3], which is for maintaining undifferentiation of and promoting proliferation of neural stem cells and/or neural progenitor cells.
[5] The method of any of the above-mentioned [1] - [4], wherein the neural stem cells and/or neural progenitor cells are derived from pluripotent stem cells.
[6] The medium of any of the above-mentioned [1] - [5], wherein the human serum albumin is a recombinant human serum albumin.
[7] The medium of any of the above-mentioned [1] - [5], wherein the human serum albumin is derived from human plasma.
[8] The medium of any of the above-mentioned [1] - [7], which comprises fatty acid in the medium.
[9] The medium of any of the above-mentioned [1] - [8], wherein the amount of the fatty acid in the medium is not more than 50 µM.
[10] The medium of any of the above-mentioned [1] - [9], wherein the amount of the fatty acid in the medium is not more than 20 µM.
[11] The medium of any of the above-mentioned [1] - [10], wherein the amount of the human serum albumin in the medium is not less than 0.2 mg/mL and not more than 20 mg/mL.
[12] The medium of any of the above-mentioned [1] - [11], wherein the amount of the human serum albumin in the medium is not less than 0.5 mg/mL and not more than 10 mg/mL.
[13] The medium of any of the above-mentioned [1] - [12], which is for suspension culture.
[14] The medium of any of the above-mentioned [1] - [13], which is a serum-free medium.
[15] The medium of any of the above-mentioned [1] - [14], which comprises basic fibroblast growth factor (bFGF).
[16] The medium of the above-mentioned [15], wherein the amount of the bFGF in the medium is not less than 1 ng/mL and not more than 200 ng/mL.
[17] A method of proliferating neural stem cells and/or neural progenitor cells, comprising adding human serum albumin to a medium.
[18] A method of maintaining undifferentiation of neural stem cells and/or neural progenitor cells, comprising adding human serum albumin to a medium.
[19] A method of promoting proliferation of neural stem cells and/or neural progenitor cells while maintaining undifferentiation thereof, comprising adding human serum albumin to a medium.
[20] The method of any of the above-mentioned [17] - [19], wherein the neural stem cells and/or neural progenitor cells are derived from pluripotent stem cells.
[21] The method of any of the above-mentioned [17] - [20], wherein the human serum albumin is recombinant human serum albumin.
[22] The method of any of the above-mentioned [17] - [20], wherein the human serum albumin is derived from human plasma.
[23] The method of any of the above-mentioned [17] - [22], wherein the medium comprises fatty acid.
[24] The method of any of the above-mentioned [17] - [23], wherein the amount of the fatty acid in the medium is not more than 50 µM.
[25] The method of any of the above-mentioned [17] - [24], wherein the amount of the fatty acid in the medium is not more than 20 µM.
[26] The method of any of the above-mentioned [17] - [25], wherein the amount of the human serum albumin in the medium is not less than 0.2 mg/mL and not more than 20 mg/mL.
[27] The method of any of the above-mentioned [17] - [26], wherein the amount of the human serum albumin in the medium is not less than 0.5 mg/mL and not more than 10 mg/mL.
[28] The method of any of the above-mentioned [17] - [27], wherein the neural stem cells and/or neural progenitor cells are cultured in suspension in the medium.
[29] A culture composition comprising the medium of any of the above-mentioned [1] - [16] and neural stem cells and/or neural progenitor cells.

### [Effect of the Invention]

According to the present invention, neural stem cells and/or neural progenitor cells can be efficiently cultured for a long term while maintaining undifferentiated state and multipotency. As a result, a large amount of neural stem cells and/or neural progenitor cells can be obtained by culturing. In addition, the cost necessary for culturing neural stem cells and/or neural progenitor cells can be reduced. According to the present invention, particularly, contamination with xenogeneic components can be avoided during culture of human neural stem cells and/or neural progenitor cells for the treatment.

### [Brief Description of the Drawings]

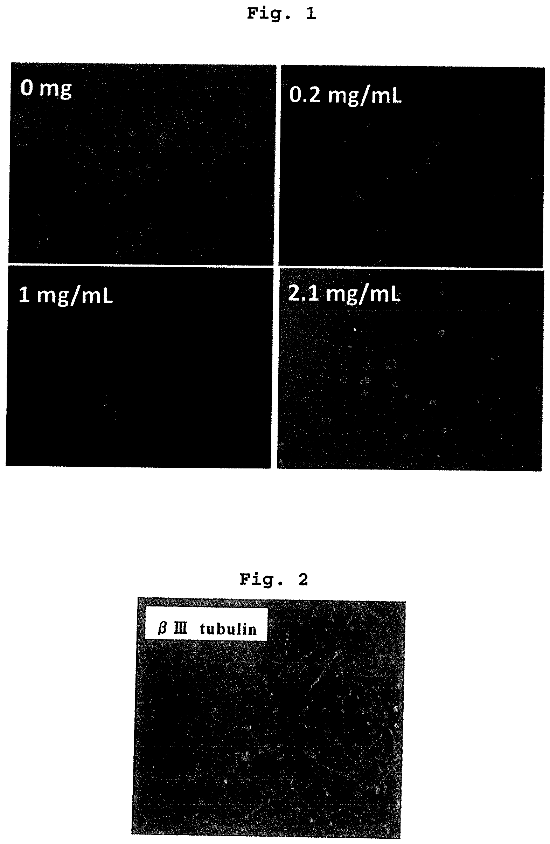

Fig. 1 shows a human serum albumin concentration-dependent suppressive tendency in the differentiation of neurosphere. Neurospheres after 14 days' culturing in a medium added with human serum albumin at a final concentration of 0.2 mg/mL, 1 mg/mL, 2.1 mg/mL are respectively shown. Neurosphere cultured in a medium added with 1 mg/mL human serum albumin (lower left) and neurosphere cultured in a medium added with 2.1 mg/mL human serum albumin (lower right) maintained good neurosphere form even after 14 days of culturing. Neurosphere cultured in a medium not added with human serum albumin (upper left) lost a neurosphere-like form after 14 days of culturing, and many cells having protrusions were observed. Neurosphere cultured in a medium added with 0.2 mg/mL human serum albumin (upper right) partly lost a neurosphere-like form after 14 days of culturing, but differentiation tended to be suppressed as compared to neurosphere cultured in a medium not added with human serum albumin (upper left).
Fig. 2 shows the results of immunostaining of neurosphere cultured in a medium added with 2.1 mg/mL human serum albumin. Many βIII tubulin (green)-positive nerve cells were observed.

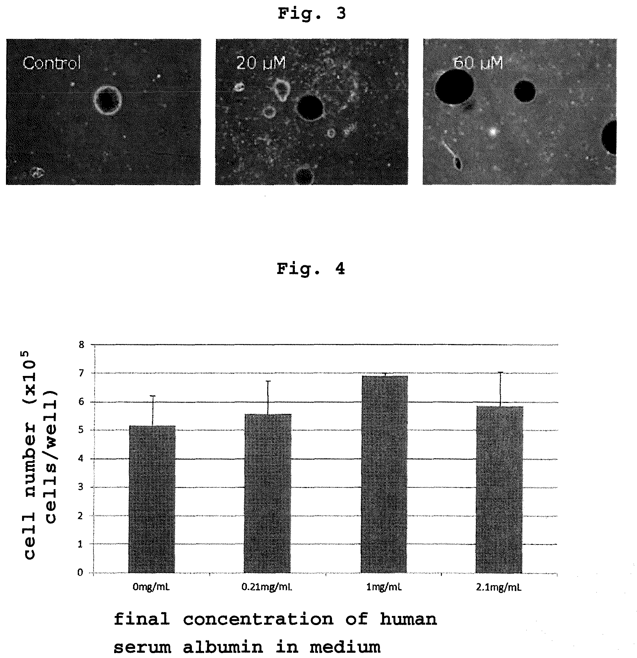

Fig. 3 shows the morphology of neurosphere cultured in a medium containing fatty acid (oleic acid) added with human serum albumin. Many black sphere images with an irregular shape were confirmed at 60 µM.
Fig. 4 shows a suppressive tendency of human serum albumin on the differentiation of Long-term self-renewing neuro epithelial-like stem cells (LtNES cells). LtNES cells after 4 - 5 days' culturing in a medium added with human serum albumin at a final concentration of 0.21 mg/mL, 1 mg/mL, 2.1 mg/mL are respectively shown. The number of cells increased most in a medium added with 1 mg/mL human serum albumin, and the cells also showed good growth in a medium added with 0.21, 2.1 mg/mL human serum albumin.

### [Description of Embodiments]

The present invention provides a medium for promoting cell proliferation of neural stem cells and/or neural progenitor cells while maintaining undifferentiated state and multipotency (hereinafter to be also referred to as the medium of the present invention), and a method of efficiently culturing neural stem cells and/or neural progenitor cells for a long term while maintaining undifferentiated state and multipotency (hereinafter to be also referred to as the method of the present invention).

### (1) Human serum albumin

Albumin is a generic term of easily coagulated proteins contained in egg albumen, serum, milk and the like. Albumin is soluble in weakly acidic to weakly alkaline solution (dilute acid, water, dilute alkali), is not salted out against 50% ammonium sulfate, and precipitated in ammonium sulfate having high concentration. It is known that many albumins having a molecular weight of not more than several tens of thousands (about 45,000) are globular proteins with isoelectric point pI 4.5 - 6. As representative ones, ovalbumin, lactalbumin, serum albumin, parvalbumin and the like can be mentioned.

Serum albumin protein is one of many proteins present in the serum, and is a soluble globular protein having a molecular weight of about 66,000. It is produced from preproalbumin via proalbumin. It is known to occupy 50% to 60% of the plasma proteins, and the concentration of serum albumin plays a significant role in the control of the osmotic pressure of the plasma and interstitial fluid. It is also known to bind to and transport poorly soluble substances (fatty acids, medicament etc.) in the blood.

The human serum albumin to be used in the present invention may be natural or non-natural human serum albumin. Human serum albumin may be one derived from plasma isolated and purified from plasma components, or recombinant human serum albumin isolated and purified from one produced by microorganisms, cells, plants and the like by gene recombination technology. When neural stem cells and/or neural progenitor cells are cultured for the purpose of treatment and the like, it is desirable that the human serum albumin to be used in the present invention is preferably a recombinant human serum albumin to avoid contamination with foreign substances.

Human serum albumin to be used in the present invention may be bonded to a metal ion such as copper ion, zinc ion and the like, glutathione, pyridoxal, bilirubin, fatty acid and the like, or may not be bonded to any of metal ion, glutathione, pyridoxal, bilirubin and fatty acid. When the medium of the present invention is produced using human serum albumin bonded to fatty acid, the amount of fatty acid carried by the human serum albumin is preferably not more than 10 mg/g, more preferably not more than 6 mg/g, and the amount of fatty acid carried by the human serum albumin may be 0 mg/g.

The amount of the fatty acid carried by human serum albumin can be measured by the methods generally practiced in the pertinent field or methods analogous thereto. Examples thereof include detection of free fatty acid by GC-MS after methyl esterification, quantification by infrared spectrum and extraction method of Duncombe, ACS-ACOD method using acyl-CoA synthetase (ACS) and acyl-CoA oxydase (ACOD) and the like. As a measurement kit, a commercially available kit can be utilized for any of them. The human serum albumin to be used in the present invention may be obtained by reducing the amount of fatty acid to be bonded to HSA having a high fatty acid-carrying amount to fall within the above-mentioned range by, for example, the method described in WO 2014/192938 and the like, and a commercially available fatty acid-free human serum albumin (e.g., Essentially fatty acid free (- 0.005%), Sigma Aldrich etc.) may also be used. To avoid contamination of the medium with a fatty acid having an indefinite composition, when human serum albumin bonded to fatty acid is used, human serum albumin to be used is preferably (1) human serum albumin having a low fatty acid-carrying amount (generally not more than 0.02%, preferably not more than 0.005%), or (2) human serum albumin obtained by carrying a fatty acid having a definite composition (e.g., linoleic acid) on the human serum albumin of (1).

Even when human serum albumin having a low fatty acid-carrying amount mentioned above is used, since human serum albumin can bind to fatty acid in the medium, the fatty acid-carrying amount of human serum albumin in the medium can be changed from the above-mentioned value.

The final glycation resultant product formed by a glycation reaction (Maillard's reaction) is known to have various toxicities, and therefore, it is preferable that human serum albumin be not a final glycation resultant product.

Human serum albumin to be used in the present invention may be in a monomer or in a multimer. Preferably, human serum albumin is a monomer.

When human serum albumin is added to the medium in the method of the present invention, the timing of addition of the human serum albumin is not particularly limited as long as the desired effects such as promotion of proliferation and maintenance of undifferentiated state of neural stem cells and/or neural progenitor cells and the like can be achieved. Human serum albumin can be added, not particularly limited as long as desired effects such as promotion of proliferation and maintenance of undifferentiated state of neural stem cells and/or neural progenitor cells and the like can be achieved, at any timing when the culture is started or during culturing.

In the present invention, the amount of human serum albumin in the medium is not particularly limited as long as desired effects such as promotion of proliferation and maintenance of undifferentiated state of neural stem cells and/or neural progenitor cells and the like can be achieved. For example, the lower limit value is not less than 0.2 mg/mL, preferably not less than 0.5 mg/mL, more preferably not less than about 1 mg/mL, most preferably not less than 1 mg/mL, and the upper limit value is not more than 20 mg/mL, preferably not more than 10 mg/mL, more preferably not more than about 5 mg/mL, most preferably not more than 5 mg/mL. Here, "about" is used to mean that ±10% is tolerable. In the present invention, the amount of human serum albumin in the medium is not particularly limited as long as desired effects such as promotion of proliferation and maintenance of undifferentiated state of neural stem cells and/or neural progenitor cells and the like can be achieved. It is 0.2 mg/mL - 20 mg/mL, preferably 0.5 mg/mL - 10 mg/mL, more preferably 1 mg/mL - 5 mg/mL, further preferably about 1 mg/mL - about 2.1 mg/mL, most preferably 1 mg/mL - 2.1 mg/mL. Here, "about" is used to mean that ±10% is tolerable. When the amount of human serum albumin contained in the medium is less than 0.2 mg/mL, it is not desirable since the ratio of the cells that differentiate into nerve cells increases. On the other hand, while higher amounts of human serum albumin contained in the medium pose no problem, generally, an amount to be contained in the medium is suitably not more than 20 mg/mL.

Human serum albumin derived from plasma can be obtained by a method known per se. For example, human serum albumin can be obtained by isolating from plasma components. While one embodiment of a method of isolating human serum albumin from human plasma components is not limited, cold ethanol fractionation (Cohn Method) can be mentioned. Cold ethanol fractionation is a method of separating plasma proteins by adjusting ethanol concentration, pH and the like under low temperature, and natural human serum albumin can be obtained from human serum albumin fraction obtained by cold ethanol fractionation (human serum albumin is fractionated in fraction V). In addition, it is possible to separate human serum albumin from plasma components by an improved method of the Cohn Method such as the method by Kistler et al (Graham, J.M., Rickwood, D. Subcellular Fractionation, a Practical Approach. Oxford University Press. 1997), the method by Tanaka K et al (Braz J Med Biol Res. 1998 Nov; 31(11): 1383-8) and the like.

Examples of the method for obtaining recombinant human serum albumin include, but are not limited to, a method including producing human serum albumin by microorganisms such as yeast and the like, animal cells or plants and the like, and isolating and purifying human serum albumin from the culture. As a method including producing human serum albumin by microorganisms, a method using a yeast (Quirk AV et al, Biotechnol Appl Biochem. 1989 Jun; 11(3): 273-87, Okabayashi K et al, J Biochem. 1991 Jul; 110(1): 103-10, JP-A-Sho60-41487, JP-A-sho63-39576, JP-A-sho63-74493), a method using Escherichia coli (Lawn RM et al, Nucleic Acids Res. 1981 Nov 25; 9(22): 6103-114), a method using Bacillus subtilis (Saunders CW et al, J Bacteriol. 1987 Jul; 169(7): 2917-25, JP-A-sho62-25133) and the like can be mentioned. As a method including producing human serum albumin by plants, a method of producing human serum albumin in the endosperm of rice (Oryza sativa) (Heet al, Proc Natl Acad Sci USA. 2011 Nov 22; 108(47): 19078-83) and the like can be mentioned. In addition, human serum albumin produced by animal cells such as CHO cell and the like can also be used. Human serum albumin is appropriately isolated and purified from these human serum albumin-producing hosts by a method selected from a method according to the above-mentioned documents, a method according to the purification method described in JP-A-hei5-317079, JP-A-hei6-56883, JP-A-hei6-245789, JP-A-hei7-170993, JP-A-hei7-170994, National Publication of International Patent Application No. hei6-500050, JP-A-2005-348745, JP-A-2007-130025 and the like, or methods known per se such as affinity chromatography, anion exchange chromatography and the like, a combination thereof and the like.

Examples of the cDNA sequence of human serum albumin include, but are not limited to, NCBI Accession Nos. AF542069, DQ986150, AY960291, NM_000477 and the like. Examples of the amino acid sequence of the human serum albumin include, but are not limited to, NCBI Accession Nos. NP_000468, AAA98797, CAA00844, CAA02034 and the like.

The human serum albumin includes
1) a protein composed of the amino sequence of the above-mentioned human serum albumin wherein one or several amino acids are deleted, substituted or added, which promotes proliferation of neural stem cells and/or neural progenitor cells,
2) a protein composed of the amino sequence of the above-mentioned human serum albumin wherein one or several amino acids are deleted, substituted or added, which shows an effect of maintaining undifferentiation of neural stem cells and/or neural progenitor cells,
and the like.

Whether the above-mentioned protein has an effect of promoting proliferation of neural stem cells and/or neural progenitor cells can be determined by, for example, seeding neural stem cells and/or neural progenitor cells in a medium added with the protein or a medium not added with the protein, measuring the number of neural stem cells and/or neural progenitor cells after 14 days' culture in the medium and comparing them, but the method is not limited thereto. The culture period can be appropriately determined such as not more than 13 days, not less than 15 days and the like. Whether the above-mentioned protein has an effect of maintaining undifferentiation of neural stem cells and/or neural progenitor cells can be determined by, for example, seeding neural stem cells and/or neural progenitor cells in a medium added with the protein or a medium not added with the protein, staining the neural stem cells and/or neural progenitor cells after 14 days' culture in the medium with the below-mentioned neural stem cell and/or neural progenitor cell marker(s), measuring the number of cells stained with the neural stem cell and/or neural progenitor cell marker(s), and comparing them, but the method is not limited thereto. The above-mentioned protein includes human serum albumin.

As the human serum albumin, a commercially available one can also be used. As a commercially available recombinant human serum albumin, products derived from recombinant rice such as Sigma-Aldrich A9731 (model number), ScienCell Research Laboratories OsrHSA-10 (model number), Wuhan Healthgen Biotechnology HY01E-10g (model number), eEnzyme HSA-1r (model number), BioVerde IBK-A1-10 (model number) and the like, and products derived from recombinant yeast such as Sigma-Aldrich A7223 (model number), A6608 (model number), A7736 (model number), Albucult (registered trade mark) (product name), Recombumin alpha (registered trade mark) (product name), AlbIX (registered trade mark) (product name) of Novozymes and the like can be mentioned. As a commercially available human serum albumin derived from human plasma, products such as Sigma-Aldrich A1887 (model number), A1653 (model number), A9511 (model number), A3782 (model number), A8763 (model number), A4327 (model number), Biological Industries Bio-Pure HSA 10% Solution (product name) and the like can be mentioned.

### (2) Neural stem cells and/or neural progenitor cells

In the present specification, neural stem cell means an undifferentiated cell maintaining multipotency into nervous system cells (nerve cells and glial cells (astrocytes, oligodendrocytes and the like), and progenitor cells thereof), and having self-replication competence. Specifically, neural stem cell is a cell having an ability to finally produce nerve cells and glial cells (astrocytes, oligodendrocytes and the like), which does not substantially produce cells other than the nervous system such as epidermal system cells, blood-lineage cells, myocytes and the like unless a special operation such as reprogramming and the like is applied. By substantially not producing means that not less than 90% of the cells produced by neural stem cells are either nerve cells and glial cells (astrocytes, oligodendrocytes and the like), or progenitor cells thereof (including neural stem cells).

In the present specification, a neural progenitor cell is an undifferentiated cell having a division potential, and capable of finally differentiating into one or more kinds of nerve cells. The neural progenitor cell refers to a cell destined to finally produce a nerve cell, which does not substantially produce anything other than nerve cells and progenitor cells thereof. A glial progenitor cell is an undifferentiated cell derived from a neural stem cell, which has a division potential, is capable of differentiating into any of astrocyte, oligodendrocyte, microglia, ependymal cell and Schwann cell, or progenitor cell thereof, and does not substantially differentiate into a nerve cell.

Since it is difficult to strictly distinguish neural stem cell from neural progenitor cell, they may be used without distinction as "neural stem cells and/or neural progenitor cells" in the present specification.

In the present invention, neural stem cells and/or neural progenitor cells derived from mammals are generally used. Examples of the mammals include, but are not limited to, rodents such as mouse, rat, hamster, guinea pig and the like, lagomorpha such as rabbit and the like, ungulata such as swine, bovine, goat, horse, sheep and the like, carnivora such as canine, feline and the like, primates such as human, monkey, cynomolgus monkey, marmoset, orangutan, chimpanzee and the like, and the like. The neural stem cells and/or neural progenitor cells to be used in the present invention are preferably neural stem cells and/or neural progenitor cells of rodents such as mouse and the like or primates such as human and the like, more preferably human neural stem cells and/or human neural progenitor cells.

Examples of the neural stem cells and/or neural progenitor cells to be used in the present invention include those derived from pluripotent stem cells, those separated from biological tissues, those directly induced to differentiate from fibroblasts and the like without intervention of pluripotent stem cells (Stem Cells. 2012 Jun; 30(6):1109-19) and the like, and are not particularly limited as long as they maintain undifferentiated state described above, maintain multipotency and maintain an ability to produce nerve cell. In the present specification, a pluripotent stem cell means an immature cell having self-replication competence and differentiation/proliferative capacity, which is a cell capable of differentiating into any tissue or cell constituting living organisms, except placenta. Examples of the pluripotent stem cell include embryonic stem cell (ES cell), induced pluripotent stem cell (iPS cell) (Takahashi K et al, Cell. 2007 Nov 30; 131(5): 861-72), spermatogonial stem cell (Kanatsu-Shinohara M et al., Biol Reprod. 2007 Jan; 76(1): 55-62), embryonic germ cell (Matsui Y et al, Cell. 1992 Sep 4; 70(5): 841-7), ES cell derived from cloned embryo obtained by nuclear transplantation (Wakayama T et al, Science. 2001 Apr 27; 292(5517): 740-3) and the like.

Neural stem cells and/or neural progenitor cells derived from pluripotent stem cells can be obtained by a method known per se. Examples of a method of producing neural stem cell and/or neural progenitor cell derived from pluripotent stem cell include a method of forming neural stem cells and/or neural progenitor cells by culturing pluripotent stem cells in suspension and performing embryoid body formation (Bain G et al, Dev Biol. 1995 Apr; 168(2): 342-57 and the like), a method including culturing pluripotent stem cells by using stromal cells and the like as feeder cells, a method including culturing pluripotent stem cells in suspension in a serum-free medium containing bFGF (Watanabe K et al, Nat Neurosci. 2005 Mar; 8(3): 288-96 and the like), a method including adhesion culture of pluripotent stem cells (ES cells etc.) in the presence of SMAD signal inhibitor Noggin and SB431542 (Chambers SM et al., Nat Biotechnol. 2009 Mar; 27(3): 275-80), a method including culturing monolayer-cultured pluripotent stem cells (ES cells etc.) in the presence of glycogen synthase kinase 3 (GSK3) inhibitor, transforming growth factor β (TGF-β) inhibitor, Notch signal inhibitor (Li W et al, Proc Natl Acad Sci USA. 2011 May 17; 108(20): 8299-304), and the like.

Preferably, the neural stem cells and/or neural progenitor cells to be used in the present invention are derived from ES cells or induced pluripotent stem cells, more preferably induced pluripotent stem cells.

Whether the cell is a neural stem cell can be confirmed by, for example, culturing the cells in suspension in a serum-free medium containing EGF and bFGF and, after a dispersion treatment of the cultured cell aggregate, subjecting the cell aggregate to adhesion culture to induce differentiation into nerve cell and glial cell.

In addition, neural stem cell can also be confirmed by a gene known to express in a neural stem cell, a transcription product thereof, a protein (neural stem cell marker) and the like.

As the neural stem cell marker, cytoskeletal protein Nestin (Science, 276, 66 (1997)), SOX1 (SRY (sex determining region Y)-box1), SOX2 (SRY (sex determining region Y)-box2), Pax6 (paired box 6), Ki67, Proliferating cell nuclear antigen (PCNA), fatty acid binding protein 7 (Fabp7, also called BLBP) and the like are known, and those of ordinary skill in the art can confirm the desired neural stem cell by appropriately combining these markers. Examples of the neural stem cells suitable for the present invention include, but are not limited to, SOX2-positive and Nestine-positive cells.

That a cell is a neural progenitor cell can be confirmed by, for example, culturing the cell, and inducing the cell to differentiate into a nerve cell.

As a gene expressed in neural progenitor cells, Tbr2 (T-box brain protein 2), MASH1 (Mammalian achaete-scute homolog 1), Nestine and the like can be mentioned. Examples of a neural progenitor cell suitable for the present invention include, but are not limited to, SOX2-negative and Nestine-positive cells.

Examples of a marker of the differentiated nerve cell include βIII tubulin, MAP2 (microtubule-associated protein) and the like.

In the present specification, maintenance of undifferentiation of neural stem cells and/or neural progenitor cells means that one or more of the cells formed by neural stem cells and/or neural progenitor cells after division continue to maintain properties of neural stem cells and/or neural progenitor cells, differentiation of neural stem cells and/or neural progenitor cells is suppressed, or neural stem cells and/or neural progenitor cells do not divide but continue to maintain properties of neural stem cells and/or neural progenitor cells. Whether the cells formed by neural stem cells and/or neural progenitor cells after division maintain properties of neural stem cells and/or neural progenitor cells can be confirmed by, for example, the aforementioned markers.

In the present specification, that differentiation of neural stem cells and/or neural progenitor cells is suppressed means that the proportion of the differentiated cells (e.g., nerve cells) in the whole cells produced by neural stem cells and/or neural progenitor cells decreases. Suppression of differentiation may be suppression of differentiation of neural stem cells and/or neural progenitor cells into nerve cells and the like. Suppression of differentiation can be confirmed by, for example, the aforementioned differentiation markers (e.g., nerve cell markers such as βIII tubulin and the like).

In one embodiment, the neural stem cells and/or neural progenitor cells to be used in the present invention are isolated. The "isolation" means that an operation to remove factors other than the object component or cell has been performed and thus it is no longer in a naturally (e.g., in vivo) occurring state.

### (3) Medium of the present invention

In one embodiment of the present invention, the present invention provides a medium for culturing neural stem cells and/or neural progenitor cells. The medium of the present invention provides effects of maintenance of undifferentiated state and multipotency of neural stem cells and/or neural progenitor cells, and promotion of proliferation thereof. In one embodiment of the present invention, the medium of the present invention is for maintaining undifferentiation of neural stem cells and/or neural progenitor cells, and for promoting proliferation of neural stem cells and/or neural progenitor cells. Alternatively, it is for maintaining undifferentiation of and promote proliferation of neural stem cells and/or neural progenitor cells.

The medium of the present invention contains human serum albumin. The human serum albumin to be added is as mentioned above. The components other than human serum albumin to be contained in the medium of the present invention are not particularly limited as long as the desired effects such as promotion of proliferation and maintenance of undifferentiated state of neural stem cells and/or neural progenitor cells and the like can be achieved, and a composition generally used for culturing neural stem cells and/or neural progenitor cells can be appropriately adopted.

The medium of the present invention may be prepared using the medium generally used for culturing animal cells as a basal medium. The basal medium is not particularly limited as long as the desired effects such as promotion of proliferation and maintenance of undifferentiated state of neural stem cells and/or neural progenitor cells and the like can be achieved. For example, media generally used for culturing animal cells such as BME medium, BGJb medium, CMRL 1066 medium, Glasgow MEM medium, Improved MEM Zinc Option medium, IMDM medium, Medium 199 medium, Eagle MEM medium, αMEM medium, DMEM medium, F-12 medium, DMEM/F12 medium, IMDM/F12 medium, ham medium, RPMI 1640 medium, Fischer's medium, or mixed medium of these and the like can be mentioned. The medium of the present invention may be prepared using the medium generally used for culturing stem cells as a basal medium. As a commercially available basal medium for culturing stem cells, RHB medium (StemCells, Inc.), TeSRTM-E6 (STEMCELL Technologies), hESF-GRO medium (NIPRO CORPORATION), HESF-DIF medium (NIPRO CORPORATION), CSTI-7 (Cell Science & Technology Institute, Inc.), Essential 6 medium (Life Technologies) and the like can be mentioned.

To avoid contamination with chemically-undefined components, the medium to be used in the present invention is preferably a medium containing chemically-defined components (Chemically defined medium; CDM).

Since serum contains components that promote differentiation of neural stem cells and/or neural progenitor cells, the medium of the present invention is preferably a serum-free medium. The "serum-free medium" in the present invention means a medium free of unconditioned or unpurified serum. In the present invention, media containing purified components derived from blood or animal tissues (e.g., growth factors such as EGF, bFGF and the like) are also included in the serum-free medium as long as unconditioned or unpurified serum is not contained.

The serum-free medium may contain a serum replacement. Examples of the serum replacement include those appropriately containing transferrin, fatty acid, collagen precursor, trace element, 2-mercaptoethanol, 3'thiolglycerol, equivalents of these and the like. Such serum replacement can be prepared by the method described in, for example, WO 98/30679. As the serum replacement, a commercially available product may also be utilized. Examples of such commercially available serum replacement include, but are not limited to, Glutamax™ (manufactured by Life Technologies), and N2 (Life Technologies Inc.).

When the medium of the present invention is used for human neural stem cells and/or neural progenitor cells, it is preferable to not contain serum albumin derived from animals other than human such as bovine serum albumin and the like, to avoid contamination with xenogeneic components.

Even when the medium of the present invention contains albumin derived from animals other than human, it is preferable to reduce the amount thereof as low as possible to avoid contamination with xenogeneic components. Specifically, the amount of albumin in the medium of the present invention, which is derived from animals other than human, is generally not more than 1000 ng/mL, preferably not more than 500 ng/mL, more preferably not more than 100 ng/mL, further preferably not more than 10 ng/mL, further more preferably 0 ng/mL.

The medium of the present invention may further contain a medium additive. Examples of the medium additive include, but are not limited to, vitamins, non-essential amino acids such asglutamine and the like, proteins such as cytokines, growth factors and the like, L-ascorbic acid, phosphoric acid L-ascorbyl magnesium, pyruvic acid sodium, 2-aminoethanol, glucose, sodium hydrogen carbonate, HEPES, insulin, progesterone, sodium selenate, putrescine and the like. Additives are preferably contained in a known concentration range.

The medium of the present invention contains essential amino acids (L-lysine, L-leucine, L-isoleucine, L-threonine, L-valine, L-phenylalanine, L-histidine, L-tryptophan). The medium of the present invention preferably contains L-serine, L-cystine, glycine, L-cysteine, L-proline, L-methionine, L-glutamic acid, L-asparagine, L-aspartic acid and L-alanine, L-glutamine, L-arginine, L-tyrosine.

The medium of the present invention contains one or more, preferably two or more, more preferably not less than 3, further preferably not less than 4, medium additives selected from the group consisting of inositol, choline chloride, folic acid, D-calcium pantothenate, thiamine (vitamin B1), pyridoxine (vitamin B6), niacinamide, vitamin B12, riboflavin (vitamin B2), D-biotin, D-glucose, pyruvic acid sodium, hypoxanthine, thymidine, lipoic acid, and putrescine hydrochloride.

The medium of the present invention preferably contains epithelial cell growth factor (EGF) and/or basic fibroblast growth factor (bFGF), more preferably bFGF.

In the present invention, while the upper limit of the amount of bFGF in the medium is not limited as long as the desired effects can be achieved, it is preferably not more than 1000 ng/ml, more preferably not more than 500 ng/ml, further preferably not more than 200 ng/ml.

In the present invention, while the lower limit of the amount of bFGF in the medium is not limited as long as the desired effects can be achieved, it is preferably not less than 0.1 ng/ml, more preferably not less than 1 ng/ml, further preferably not less than 10 ng/ml.

In the present invention, while the amount of bFGF in the medium is not limited as long as the desired effects can be achieved, it is preferably 0.1 ng/ml - 1000 ng/ml, more preferably 1 ng/ml - 200 ng/ml, further preferably 10 ng/ml - 200 ng/ml.

In one embodiment, the medium of the present invention contains bFGF (final concentration 10 ng/ml - 200 ng/ml).

In addition, the medium of the present invention is preferable substantially free of a substance having an effect of promoting differentiation of neural stem cells and/or neural progenitor cells (to be also referred to as neuronal differentiation promoting substance in the present specification).

Examples of the neuronal differentiation promoting substance include BDNF (Brain-derived neurotrophic factor), GDNF (Glial cell line-derived neurotrophic factor), cAMP (Cyclic adenosine monophosphate), dbcAMP (dibutyryl cAMP), DAPT (tert-butyl (2S)-2-[[(2S)-2-[[2-(3,5-difluorophenyl)acetyl]amino]propanoyl]amino]-2-phenylacetate), compound E (N-[(1S)-2-[[(3S)-2,3-Dihydro-1-methyl-2-oxo-5-phenyl-1H-1, 4-benzodiazepin-3-yl]amino]-1-methyl-2-oxoethyl]-3,5-difluorobenzeneacetamide), SU5402 (2-[(1,2-Dihydro-2-oxo-3H-indol-3-ylidene)methyl]-4-methyl-1H-pyrrole-3-propanoic acid), SU6668 (3-[2,4-dimethyl-5-[(E)-(2-oxo-1H-indol-3-ylidene)methyl]-1H-pyrrol-3-yl]propanoic acid; Orantinib; and 3-[2,4-dimethyl-5-[(E)-(2-oxo-1H-indol-3-ylidene)methyl)-1H-pyrrol-3-yl] propanoic acid).

Being substantially free of a neuronal differentiation promoting substance means that even when a neuronal differentiation promoting substance is contained, its amount cannot promote differentiation of neural stem cells and/or neural progenitor cells, and the amount is appropriately determined according to the kind of the neuronal differentiation promoting substance to be used.

More preferably, the concentration of the neuronal differentiation promoting substance contained in the medium of the present invention is 0 µM.

The medium of the present invention may contain a fatty acid. Examples of the fatty acid to be contained in the medium of the present invention include, but are not limited to, oleic acid, linoleic acid, α-linolenic acid, γ-linolenic acid, palmitic acid, stearic acid, arachidonic acid, icosapentaenoic acid, docosahexaenoic acid, butyric acid, acetic acid, pulmitoleic acid, valeric acid (valerianic acid), caproic acid, enanthic acid (hepthylic acid), caprylic acid, pelargric acid, capric acid, lauric acid, myristic acid, pentadecylic acid, margaric acid, Khusenic acid, eleostearic acid, arachidic acid, 8,11- eicosadienoic acid, 5,8,11-eicosatrienoic, behenic acid, lignoceric acid, nervonic acid, cerotic acid, montanic acid, melissic acid and the like. The fatty acid to be contained in the medium of the present invention is not particularly limited as long as the desired effects can be achieved, and may be saturated fatty acid or unsaturated fatty acid.

While not particularly limited as long as the desired effects can be achieved, linoleic acid is generally used for the medium.

The medium of the present invention generally contains fatty acid derived from the basal medium.

In the present invention, the amount of fatty acid in the medium is not particularly limited as long as the desired effects such as promotion of proliferation and maintenance of undifferentiated state of neural stem cells and/or neural progenitor cells and the like can be achieved, or an adverse influence is not exerted on the desired effects. It is not more than 50 µM, preferably not more than 25 µM, more preferably not more than 22 µM, further preferably not more than 20 µM, further more preferably less than 20 µM. When the concentration of fatty acid is 60 µM, it is not desirable since many of neural stem cells and/or neural progenitor cells become cells that form black sphere with an irregular shape and having lower proliferative capacity than normal. The amount of fatty acid in the medium is desirably not more than 50 µM, and a smaller amount is more desirable since black sphere with an irregular shape is more difficult to form.

The medium of the present invention preferably contains fatty acid in an amount contained in general basal media, and the amount thereof is preferably not less than 0.01 µM, more preferably not less than 0.05 µM, further preferably not less than 0.1 µM.

The concentration of the fatty acid in the medium of the present invention is not limited as long as the desired effects can be achieved. It is preferably 0.01 µM - 50 µM, more preferably 0.05 µM - 25 µM, further preferably 0.05 µM - 22 µM, further more preferably 0.1 µM - 22 µM, most preferably 0.1 µM - 20 µM.

When the medium of the present invention contains human serum albumin bonded to fatty acid, the above-mentioned "amount of fatty acid in the medium" contains, in addition to the free fatty acid in the medium, fatty acid bonded to human serum albumin.

When the medium of the present invention is produced by adding human serum albumin bonded to fatty acid, it is desirable to produce the medium of the present invention by using human serum albumin having an appropriate fatty acid-carrying amount so that the desired amount of fatty acid in the medium can be achieved.

When two or more kinds of fatty acids are contained in the medium of the present invention, the total amount thereof is preferably set to fall within the above-mentioned range.

Preferably, the medium of the present invention is a serum-free medium containing bFGF (10 ng/mL - 200 ng/mL) and human serum albumin (0.5 mg/mL - 10 mg/mL), and having an amount of fatty acid in the medium of 0.05 µM - 50 µM.

More preferably, the medium of the present invention is a serum-free medium containing bFGF (10 ng/mL - 200 ng/mL) and human serum albumin (about 1 mg/mL - about 2.1 mg/mL), wherein the concentration of serum albumin derived from animal other than human is not more than 500 ng/mL, and the amount of fatty acid in the medium is about 0.1 µM - about 20 µM. Here, "about" is used to mean that ±10% is tolerable.

In one preferable embodiment, the medium of the present invention contains transferrin, insulin, NaHCO₃, selenium, ethanolamine, bFGF in addition to human serum albumin. More preferably, the medium of the present invention is a serum-free medium containing, in addition to human serum albumin, transferrin, insulin, NaHCO₃, selenium, ethanolamine, bFGF, inositol, choline chloride, folic acid, D-calcium pantothenate, thiamine (vitamin B1), pyridoxine (vitamin B6), niacinamide, vitamin B12, riboflavin (vitamin B2), D-biotin, D-glucose, pyruvic acid sodium, hypoxanthine, thymidine, lipoic acid, putrescine, linoleic acid, L-lysine, L-leucine, L-isoleucine, L-threonine, L-valine, L-phenylalanine, L-histidine, L-tryptophan and L-asparagine.

In one embodiment, the medium of the present invention is prepared with DMEM/F-12 medium as a basal medium, and contains transferrin (0.5 µg/mL - 100 µg/mL), insulin (5 µg/ml - 1 mg/ml), NaHCO₃ (100 µg/ml - 5 mg/ml), sodium selenate (2 ng/ml - 1 µg/ml), ethanolamine (100 ng/ml - 100 µg/ml), and bFGF (10 ng/ml - 200 ng/ml), as well as human serum albumin (0.5 mg/mL - 10 mg/mL).

In another preferable embodiment, the medium of the present invention contains, in addition to human serum albumin, bFGF, hLIF, glucose, glutamine, NaHCO₃, HEPES, insulin, transferrin, progesterone, sodium selenate, and putrescine. More preferably, the medium of the present invention is a serum-free medium containing, in addition to human serum albumin, glucose, glutamine, NaHCO₃, HEPES, insulin, transferrin, progesterone, sodium selenate, putrescine, inositol, choline chloride, folic acid, D-calcium pantothenate, thiamine(vitamin B1), pyridoxine (vitamin B6), niacinamide, vitamin B12, riboflavin (vitamin B2), D-biotin, D-glucose, pyruvic acid sodium, hypoxanthine, thymidine, lipoic acid, putrescine, linoleic acid, L-lysine, L-leucine, L-isoleucine, L-threonine, L-valine, L-phenylalanine, L-histidine, L-tryptophan, and L-asparagine.

In one embodiment, the medium of the present invention uses DMEM/F-12 medium as a basal medium, and contains bFGF (10 ng/mL - 200 ng/mL), hLIF (1 ng/mL - 100 ng/mL), glucose (1 mg/mL - 10 mg/mL), glutamine (100 µg/mL - 1 mg/mL), NaHCO₃ (100 µg/mL - 5 mg/mL), HEPES (100 µg/mL - 5 mg/mL), insulin (5 µg/mL - 1 mg/mL), transferrin (0.5 µg/mL - 100 µg/mL), progesterone (2 ng/mL - 1 µg/mL), sodium selenate (2 ng/mL - 1 µg/mL), putrescine (100 µg/mL - 10 mg/mL), as well as human serum albumin (0.5 mg/mL - 10 mg/mL).

The medium of the present invention can be used for any culture method such as adhesion culture, suspension culture, embedded culture, tissue culture and the like. Preferably, this medium is for suspension culture.

The medium of the present invention can be preferably used for culturing neural stem cells and/or neural progenitor cells derived from any animals. The neural stem cells and/or neural progenitor cells that can be cultured using the medium of the present invention include neural stem cells and/or neural progenitor cells derived from, for example, rodents such as mouse, rat, hamster, guinea pig and the like, lagomorpha such as rabbit and the like, ungulata such as swine, bovine, goat, horse, sheep and the like, carnivora such as canine, feline and the like, primates such as human, monkey, cynomolgus monkey, marmoset, orangutan, chimpanzee and the like, and the like, preferably, neural stem cells and/or neural progenitor cells derived from human.

### (4) Method of the present invention

In one embodiment of the present invention, the present invention provides a method of culturing neural stem cells and/or neural progenitor cells, which comprises adding human serum albumin to a medium. The method is also a method of proliferating neural stem cells and/or neural progenitor cells and maintaining undifferentiation thereof.

In one embodiment of the present invention, the method of the present invention includes a step of culturing neural stem cells and/or neural progenitor cells in the medium of the present invention. In another embodiment of the present invention, the method of the present invention includes a step of adding human serum albumin to a medium free of human serum albumin and culturing in the presence of the human serum albumin for a certain period.

While the period of culturing neural stem cells and/or neural progenitor cells in the method of the present invention is not particularly limited as long as the desired effects such as promotion of proliferation and maintenance of undifferentiated state of neural stem cells and/or neural progenitor cells and the like can be achieved, it is generally not less than 2 days, preferably not less than 4 days, further preferably not less than 8 days.

When neural stem cells and/or neural progenitor cells are cultured for not less than 4 consecutive days, the medium is preferably exchanged once in 3 days, preferably once in 2 days.

While the time of addition of the human serum albumin to the medium in the method of the present invention is not particularly limited as long as it is such length of time that can achieve the desired effects such as promotion of proliferation and maintenance of undifferentiated state of neural stem cells and/or neural progenitor cells and the like, it is preferable to perform culturing in a medium containing human serum albumin throughout the entire culture period. The composition of the medium is as described above.

In the method of the present invention, neural stem cells and/or neural progenitor cells can be cultured according to a known method such as adhesion culture, suspension culture, tissue culture and the like, except that the medium to be used contains human serum albumin. The culture method can be appropriately selected according to the object. Examples of the adhesion culture method of neural stem cells and/or neural progenitor cells include the methods described in Flanagan LA et al, J Neurosci Res. 2006 Apr; 83(5): 845-56, Conti L et al, PLoS Biology., 2005 Sep; 3(9): e283 and the like. Suspension culture of neural stem cells and/or neural progenitor cells refers to culturing neural stem cells and/or neural progenitor cells under the condition under which they are non-adhesive to an incubator or feeder cells (when used) in the medium. Examples of the suspension culture method of neural stem cells and/or neural progenitor cells include neurosphere method (Reynolds BA and Weiss S., Science, USA, 1992 Mar 27; 255(5052): 1707-10), Serum-free Suspension culture of Embryoid Bodies-like aggregates method (SFEB method, SFEBq method; Watanabe et al, Nature Neuroscience 8, 288-296 (2005)) and the like. Tissue culture of neural stem cells and/or neural progenitor cells is a method of culturing a tissue containing neural stem cells and/or neural progenitor cells as a tissue section such as slice and the like or the whole tissue. Examples of the tissue culture of neural stem cells and/or neural progenitor cells include slice culture methods described in O'Rourke NA et al, Science. 1992 Oct 9; 258(5080): 299-302., Komuro H et al, Science. 1992 Aug 7; 257(5071): 806-9 and the like. In the method of the present invention, neural stem cells and/or neural progenitor cells are preferably cultured in suspension in a medium containing human serum albumin.

By suspension culture, neural stem cells and/or neural progenitor cells form a spherical mass, so-called neurosphere. When suspension culture is performed, the presence or absence and the level of proliferation of neural stem cells and/or neural progenitor cells can be evaluated by measuring the size of the neurosphere to be formed, or the number of cells constituting the neurosphere. In addition, the presence or absence and the level of proliferation of neural stem cells and/or neural progenitor cells can also be evaluated by measuring the number of viable cells by using a cell staining reagent such as Trypan Blue and the like.

In the method of the present invention, an incubator to be used for culturing neural stem cells and/or neural progenitor cells is not particularly limited as long as the neural stem cells and/or neural progenitor cells can be cultured. Examples thereof can include flask, tissue culture flask, dish, petri dish, tissue culture dish, multidish, microplate, microwell plate, multiplate, multiwell plate, microslide, chamber slide, schale, tube, tray, culture bag, and roller bottle.

An incubator used for culturing neural stem cells and/or neural progenitor cells may be cell adhesive or cell non-adhesive, and is appropriately selected according to the object. When neural stem cells and/or neural progenitor cells are cultured by suspension culture, the incubator is preferably cell adhesive to remove cells susceptible to differentiation.

When neural stem cells and/or neural progenitor cells are cultured by adhesion culture, the incubator is preferably cell adhesive. A cell adhesive incubator may be coated with any cell supporting substrate such as extracellular matrix (ECM) and the like or an artificial material mimicing the function thereof, for the purpose of improving the adhesiveness of the cells to the surface of the incubator. The cell supporting substrate may be any substance aiming at adhesion of stem cells or feeder cells (when used).

Other culture conditions can be appropriately determined. For example, the culture temperature is not particularly limited as long as the desired effects such as promotion of proliferation and maintenance of undifferentiated state of neural stem cells and/or neural progenitor cells and the like can be achieved. It is about 30 - 40°C, preferably about 37°C. The CO₂ concentration is about 1 - 10%, preferably about 2 - 5%. The oxygen concentration is generally 1 - 40%, and is appropriately selected according to culture conditions and the like.

### (5) Culture composition comprising medium containing human serum albumin and neural stem cells and/or neural progenitor cells

The present invention further provides a culture composition comprising the above-mentioned medium of the present invention and neural stem cells and/or neural progenitor cells (also referred to as the culture composition of the present invention in the present specification). The culture composition includes a resultant product obtained by culturing the cells. The definition and embodiment of each term relating to the culture composition of the present invention are the same as those described above.

The neural stem cells and/or neural progenitor cells in the culture composition of the present invention are cells that are viable and proliferative.

The purity of the neural stem cells and/or neural progenitor cells in the culture composition of the present invention (percentage of the number of neural stem cells and/or neural progenitor cells in the total number of cells) is generally not less than 70%, preferably not less than 80%, more preferably not less than 90%, further preferably not less than 99%, most preferably 100%.

In the culture composition of the present invention, neural stem cells and/or neural progenitor cells are present in the medium of the present invention. In one embodiment, the culture composition of the present invention is a suspension of neural stem cells and/or neural progenitor cells in the medium of the present invention. The culture composition of the present invention may be sealed in an appropriate container.

In one embodiment, the culture composition of the present invention can be provided in a cryopreserved state. The culture composition of the present invention can be cryopreserved, and can be used by thawing and raising from sleep as necessary. For cryopreservation, a known cell cryopreservation method can be used. As an example of cryopreservation, the method where dimethyl sulfoxide is added to the culture composition of the present invention, and the culture composition of the present invention is preserved at - 80 - -200°C, preferably -196°C (in liquid nitrogen) can be mentioned.

While the present invention is explained in more detail in the following by referring to Examples, they do not limit the scope of the present invention.

### [Examples]

### Example 1: Functional evaluation of human serum albumin

### (1) Induction of neurosphere from human pluripotent stem cells

Media hormone mix (MHM) medium added with B27 supplement (1x; Life Technologies), bFGF (PeproTech inc.), hLIF (Millipore), Y27632 (Wako Pure Chemical Industries, Ltd.) was produced, and iPS cells dispersed in TrypLE™ Select to single cells were cultured in suspension in the medium under 37°C, 5% CO₂, 4% O₂ environment. The medium was exchanged every 7 days. After culturing, neural stem cells and/or neural progenitor cells formed neurosphere which is a spherical cell aggregate.

### (2) Culture method of neurosphere

Media hormone mix (MHM) medium (Life Technologies) added with B27 supplement (1x; Life Technologies), bFGF (final concentration 20 ng/mL; PeproTech inc.), hLIF (final concentration 10 ng/mL; Millipore) was produced, and neural stem cells and/or neural progenitor cells induced from human pluripotent stem cells were cultured in suspension in the medium under 37°C, 5% CO₂, 4% O₂ environment. The medium was exchanged every 7 days. After culturing, neural stem cells and/or neural progenitor cells formed neurosphere which is a spherical cell aggregate.

TrypLE Select (Life Technologies) was used for passaging the neurosphere. The medium was removed from the culture dish during neurosphere culture and replaced with TrypLE Select. The cells were incubated in TrypLE Select at 37°C for 10 min, and thereafter pipetted to give single cells. The dispersed cells were seeded in the above-mentioned medium at 1.0x10⁵ cells/mL, and cultured in suspension in the medium under 37°C, 5% CO₂ environment.

### (3) Functional evaluation of human serum albumin

Using the neurosphere cultured in (2), functional evaluation of the medium was performed. Media hormone mix (MHM) medium (Life Technologies) added with bFGF (final concentration 20 ng/mL) and hLIF (final concentration 10 ng/mL) was produced and used as a test basal medium. Neurosphere was cultured in the test basal medium added with human serum albumin (Essentially fatty acid free (- 0.005%), Sigma Aldrich) at a final concentration of 0.2, 1 or 2.1 mg/mL (human serum albumin-added medium) or a medium without the addition (control medium), and functional evaluation of the human serum albumin was performed. The medium was exchanged every 7 days and, after 14 days' culturing, the morphology of the neurosphere formed was observed under a microscope. The results are shown in Fig. 1. (Before the start of the culture in (3), the medium used before culturing in (3) was sufficiently diluted and removed to reduce incorporation of the medium as much as possible.)

The neurosphere cultured in the control medium lost a spherical form after 14 days' culturing, and showed a cell form with an elongated protrusion seen in differentiated nerve cells. On the other hand, the neurospheres cultured in 1 mg/mL human serum albumin-added medium, 2.1 mg/mL human serum albumin-added medium maintained the spherical form even after 14 days' culturing, and the size became larger as compared to that before culturing. The neurospheres cultured in these media did not show a cell form with an elongated protrusion seen in differentiated cells. The neurosphere cultured in 0.2 mg/mL human serum albumin-added medium showed a cell form with an elongated protrusion seen in a part of the differentiated cells. However, the number of protrusion was small as compared to the control medium and many neurospheres maintaining the spherical form were also observed.

From the results, human serum albumin concentration-dependent differentiation suppressive tendency on the neurosphere was shown. When the amount of human serum albumin is not less than 0.2 mg/mL, many neurospheres were observed to maintain the spherical form. Particularly, when human serum albumin was not less than 1 mg/mL, formation of a good neurosphere image was confirmed.

### Example 2: Differentiation induction into nerve cell

It is demonstrated that neurosphere cultured in human serum albumin-added medium maintains the properties as neural stem cells and/or neural progenitor cells.

Similar to Example 1, neurosphere derived from human pluripotent stem cells was cultured in 2.1 mg/mL human serum albumin-added medium for 14 days, and differentiation induction culture was performed. For the differentiation induction culture, the neurosphere cultured in human serum albumin-added medium was subjected to a dispersion treatment. TrypLE Select (Life Technologies) was used for the dispersion treatment. The medium was removed from the culture dish during neurosphere culture and replaced with TrypLE Select. The cells were incubated in TrypLE Select at 37°C for 10 min, and thereafter pipetted to give single cells. The number of the cells was measured using a hemocytometer. The dispersed cells were seeded in a 48 well plate, coated with poly-L-ornithine/fibronectin, at 1.5x10⁵ cells/well, and cultured in Media hormone mix (MHM) medium (Life Technologies) added with B27 supplement (1x; Life Technologies) for 20 days. Culturing was performed under 37°C, 5% CO₂ environment, and the medium was exchanged every two days.

After culturing, the cells were fixed and immunostained using antibody against βIII tubulin as a nerve cell marker. The results are shown in Fig. 2.

The cells subjected to differentiation induction culture contained many βIII tubulin positive nerve cells. It was shown that neurosphere cultured in human serum albumin-added medium can efficiently differentiate into nerve cells.

### Example 3: Functional evaluation of fatty acid

Fatty acid was further added to human serum albumin-added medium, and function evaluation was performed. An object of Example 3 is to confirm the action of human serum albumin with a high fatty acid-carrying amount.

Similar to Example 1, 2.1 mg/mL human serum albumin-added medium was produced. A medium was produced by adding oleic acid (Tokyo Chemical Industry Co., Ltd.) at a final concentration of 20 µM or 60 µM to this medium, and a medium without addition of oleic acid was produced, and neurospheres were cultured in these media. (A medium containing fatty acid and human serum albumin can also be produced by mixing fatty acid with a given concentration of human serum albumin solution in advance, and adding the mixture to the medium.) Almost 100% of oleic acid in the medium was assumed to have bonded to HSA. The medium was exchanged every 7 days and, after 14 days' culturing, the morphology of the neurosphere formed was observed under a microscope. The results are shown in Fig. 3.

The neurosphere cultured in 20 µM oleic acid-added medium showed a good neurosphere form similar to that of neurosphere cultured in the medium without addition of fatty acid. On the other hand, many neurospheres cultured in 60 µM oleic acid-added medium were confirmed to show a black sphere with an irregular shape.

### Example 4: Functional evaluation of human serum albumin

### (1) Long-term self-renewing neuro epithelial-like stem cells (hereinafter LtNES cells) induction method

EB was formed from iPS cells, and cultured in a medium obtained by adding transferrin (final concentration 0.5 - 10 µg/ml) and ethanolamine (final concentration 5 - 50 µM) to a medium corresponding to a composition of E6 medium (Life Technologies or STEMCELL Technologies) without ascorbic acid and transferrin for 4 days. EB was seeded in a dish coated with poly-L-ornithine (PO), cultured for about 10 days in the above-mentioned medium, and formation of a rosette-like structure was confirmed. The rosette part was removed, and subjected as neurosphere to suspension culture in the above-mentioned medium for about 7 days. The neurosphere was dispersed in trypsin/EDTA, and cultured in the above-mentioned medium on a dish coated with PO/laminin to give LtNES cells. The LtNES cells are composed of a mixture of neural stem cells and neural progenitor cells.

The above-mentioned E6 medium (Essential 6 medium) is produced by an E8 medium-based production method and does not contain bFGF and TEPβ as described in the homepage of Life Technologies <URL:http://www.lifetechnologies.com/order/catalog/product/A151 6401>. E8 medium (Essential 8 medium) is described in Nat Methods 2011 May; 8(5):424-429).

The components of the above-mentioned E6 medium are also described in the summary of Stem Cells. 2014 Apr; 32(4), 1032-42.

### (2) Functional evaluation of human serum albumin

Transferrin (final concentration 0.5 - 10 µg/ml), ethanolamine (final concentration 5 - 50 µM) and bFGF (final concentration 5 - 100 ng/ml) were added to a medium corresponding to a composition of E6 medium (Life Technologies or STEMCELL Technologies) without ascorbic acid and transferrin to produce a test basal medium.

Albumin derived from human serum (Sigma Aldrich) was added to the test basal medium at a final concentration of 0, 0.21, 1, 2.1 mg/ml to give test media.

Using each test medium, LtNES cells (1.5×10⁵ cells) induced from human pluripotent stem cells were cultured. The cells were cultured in an incubator at 37°C, 5% CO₂ atmosphere. The medium was exchanged every two days, and the cells were cultured for 4 - 5 days. After culturing, cell dispersion treatment was performed by adding TrypLE Select instead of the test medium and then incubating it at 37°C for 1 min. TrypLE Select was diluted with the medium, pipetting was performed to give single cells, and the number of the cells was counted and evaluated. Dead cells were stained with Trypan Blue (Life Technologies Inc.) and the cell number was measured by hemocytometer.

The results are shown in Fig. 4. The cell number increased most in 1 mg/ml human serum albumin -added medium and the cells showed good proliferation also in 0.21, 2.1 mg/ml human serum albumin -added medium.

### [Industrial Applicability]

According to the present invention, cell proliferation can be promoted while maintaining undifferentiated state and multipotency of neural stem cells and/or neural progenitor cells, and the personnel costs and economical costs necessary for culturing neural stem cells and/or neural progenitor cells can be reduced.

The contents disclosed in any publication stated in the present specification, including patents, patent applications and scientific literatures, are hereby incorporated in their entireties by reference, to the extent that they have been disclosed herein.

This application is based on a patent application No. 2015-069979 filed in Japan (filing date: March 30, 2015), the contents of which are incorporated in full herein.

## Claims

1. A medium for neural stem cells and/or neural progenitor cells, comprising human serum albumin.

2. The medium according to claim 1, which is for maintaining undifferentiation of neural stem cells and/or neural progenitor cells.

3. The medium according to claim 1 or 2, which is for promoting proliferation of neural stem cells and/or neural progenitor cells.

4. The medium according to any one of claims 1 to 3, which is for maintaining undifferentiation of and promoting proliferation of neural stem cells and/or neural progenitor cells.

5. The method according to any one of claims 1 to 4, wherein the neural stem cells and/or neural progenitor cells are derived from pluripotent stem cells.

6. The medium according to any one of claims 1 to 5, wherein the human serum albumin is a recombinant human serum albumin.

7. The medium according to any one of claims 1 to 5, wherein the human serum albumin is derived from human plasma.

8. The medium according to any one of claims 1 to 7, which comprises fatty acid in the medium.

9. The medium according to any one of claims 1 to 8, wherein the amount of the fatty acid in the medium is not more than 50 µM.

10. The medium according to any one of claims 1 to 9, wherein the amount of the fatty acid in the medium is not more than 20 µM.

11. The medium according to any one of claims 1 to 10, wherein the amount of the human serum albumin in the medium is not less than 0.2 mg/mL and not more than 20 mg/mL.

12. The medium according to any one of claims 1 to 11, wherein the amount of the human serum albumin in the medium is not less than 0.5 mg/mL and not more than 10 mg/mL.

13. The medium according to any one of claims 1 to 12, which is for suspension culture.

14. The medium according to any one of claims 1 to 13, which is a serum-free medium.

15. The medium according to any one of claims 1 to 14, which comprises basic fibroblast growth factor (bFGF).

16. The medium according to claim 15, wherein the amount of the bFGF in the medium is not less than 1 ng/mL and not more than 200 ng/mL.

17. A method of proliferating neural stem cells and/or neural progenitor cells, comprising adding human serum albumin to a medium.

18. A method of maintaining undifferentiation of neural stem cells and/or neural progenitor cells, comprising adding human serum albumin to a medium.

19. A method of promoting proliferation of neural stem cells and/or neural progenitor cells while maintaining undifferentiation thereof, comprising adding human serum albumin to a medium.

20. The method according to any one of claims 17 to 19, wherein the neural stem cells and/or neural progenitor cells are derived from pluripotent stem cells.

21. The method according to any one of claims 17 to 20, wherein the human serum albumin is recombinant human serum albumin.

22. The method according to any one of claims 17 to 20, wherein the human serum albumin is derived from human plasma.

23. The method according to any one of claims 17 to 22, wherein the medium comprises fatty acid.

24. The method according to any one of claims 17 to 23, wherein the amount of the fatty acid in the medium is not more than 50 µM.

25. The method according to any one of claims 17 to 24, wherein the amount of the fatty acid in the medium is not more than 20 µM.

26. The method according to any one of claims 17 to 25, wherein the amount of the human serum albumin in the medium is not less than 0.2 mg/mL and not more than 20 mg/mL.

27. The method according to any one of claims 17 to 26, wherein the amount of the human serum albumin in the medium is not less than 0.5 mg/mL and not more than 10 mg/mL.

28. The method according to any one of claims 17 to 27, wherein the neural stem cells and/or neural progenitor cells are cultured in suspension in the medium.

29. A culture composition comprising the medium according to any of claims 1 to 16 and neural stem cells and/or neural progenitor cells.
